# FASCICULE DE BREVET EUROPEEN

(11) **EP 3 247 466 B1**
(45) Date de publication et mention de la délivrance du brevet: **03.04.2019**
(21) Numéro de dépôt: 15801157.7
(22) Date de dépôt: 26.11.2015
(51) Int. Cl.: A61K 8/9783, A61Q 19/00, A61Q 19/08

(54) **EXTRAIT PEPTIDIQUE ET OSIDIQUE DE FRUIT DE SCHIZANDRA ET AMELIORATION DE LA REPONSE DU SYSTEME NEUROSENSORIEL CUTANE**
PEPTID UND GLYKOSIDEXTRAKT AUS DER SCHISANDRA-FRUCHT UND VERBESSERUNG DER REAKTION DES KUTANEN NEUROSENSORISCHEN SYSTEMS
PEPTIDE AND GLYCOSIDE EXTRACT OF SCHIZANDRA FRUIT AND IMPROVEMENT IN THE RESPONSE OF THE CUTANEOUS NEUROSENSORY SYSTEM

(30) Priorité: 26.11.2014 FR 1461518
(43) Date de publication de la demande: 29.11.2017
(73) Titulaire: Laboratoires Expanscience, 92048 Paris La Défense Cedex (FR)
(72) Inventeur: MSIKA, Philippe, 78000 Versailles (FR); BAUDOUIN, Caroline, 78120 Rambouillet (FR); BREDIF, Stéphanie, 28210 Croisilles (FR)
(74) Mandataire: Regimbeau
(86) Numéro de dépôt international: PCT/EP2015/077795
(87) Numéro de publication internationale: WO 2016/083515

(56) Documents cités:
- WO-A2-2011/012612
- JP-A- H11 180 885
- JP-A- 2000 178 168
- JP-A- 2002 241 293
- US-A1- 2012 052 142
- ONIONS A ET AL: "A TASTE OF THE ORIENT", INTERNET CITATION, mars 1992 (1992-03), XP002945803, Extrait de l'Internet: URL:HTTP://WWW.CAMPO-RESEARCH.COM/CAMPO/PR ESS/TASTE2.HTML [extrait le 2001-03-15]

## Description

### DOMAINE DE L'INVENTION

La présente invention concerne l'utilisation d'un extrait peptidique et osidique de fruit de Schizandra en tant qu'agent neurosensoriel cutané, notamment pour améliorer la réponse du système neurosensoriel cutané. Une telle utilisation est avantageusement cosmétique.

### ART ANTÉRIEUR

Il existe dans le monde à peu près 25 espèces appartenant au genre *Schizandra.* Environ seize d'entre elles sont chinoises. Ces arbustes sont originaires du Nord de la Chine et des régions adjacentes de Russie et Corée.

*Schizandra* est une plante dioïque (pieds aux fleurs mâles et femelles distincts). Le fruit se présente sous forme de grappe pendante qui rappelle un peu celle du groseillier. Elle est pourvue d'un pédoncule nu dans sa partie supérieure (environ 5-10 cm) qui est recouvert dans sa partie inférieure par des baies rouges vifs, un peu plus grosses, plus compactes et plus fermes que la groseille. La graine, sphérique, mesure quelques millimètres.

Deux espèces sont officiellement reconnues comme médicinales en Chine, *S. chinensis* et *S. sphenanthera.* Leurs baies sont utilisées en médecine traditionnelle chinoise pour le traitement de la toux, de l'asthme, des sueurs nocturnes, des éjaculations nocturnes et de la diarrhée chronique. Elles sont aussi utilisées comme tonifiants et pour le traitement de la fatigue chronique.

De nom botanique Schizandra, cette plante appartient à la classe des Magnoliopsida et à l'ordre des Magnoliales. La famille botanique est celle des Schisandracea. Le terme Schizandra utilisé tel quel peut désigner les deux plantes différentes que sont Schizandra chinensis et Schizandra sphenanthera. Longtemps ces deux espèces ont été considérées comme équivalentes, elles pouvaient être désignées selon leur origine « Northern Schizandra » pour Schizandra chinensis et « Southern Schizandra » pour Schizandra sphenanthera.

Schizandra chinensis est aussi appelée pinyin, wǔ wèi zi, littéralement « baie à 5 parfums ». Schizandra chinensis est une liane arborescente caduque sauvage à croissance lente pouvant atteindre 9 à 10 mètres de hauteur.

Schizandra sphenanthera Rehd. et Wils. est aussi appelée : schisandre à fleurs orangées (en France), southern Schizandra, lemon wood (en Angleterre), hua zhong wu wei zi, nan wu wie zi (en Chine).

Si le fruit de *S. Chinensis* a été l'objet d'une littérature conséquente, c'est beaucoup moins le cas de *S. sphenanthera.* Plusieurs motifs expliquent ceci :
- une moindre réputation quant à son utilisation selon la tradition chinoise,
- une quasi-absence d'usage en occident,
- une moindre teneur en néo-lignanes totaux comparée à *S. chinensis.*

L'une des différences majeures entre les deux espèces porte sur la nature et les teneurs des néo-lignanes contenus dans leurs fruits respectifs. Cette sous-famille de constituants chimiques représente la grande originalité du genre *Schizandra.* L'article (Huyke et al., 2007) a rapporté une étude comparative des effets sur la prolifération cellulaire d'extraits de *S. chinensis* et *S. sphenanthera.*

Il est généralement considéré que *S. sphenanthera* est inférieur à *S. chinensis* au plan médicinal et qu'il n'est utile qu'en tant que source alternative des lignanes actifs.

Sur un plan commercial, le fruit de *S. sphenanthera* est considéré comme moins coûteux que celui de *S. chinensis.*

### Caractéristiques des fruits

Le fruit sec de *Schizandra* comprend environ 20% d'huiles essentielles dont 7 à 30 % d'insaponifiables (Huyke et al., 2007). Les lignanes sont compris dans la fraction insaponifiable des huiles. D'autres constituants actifs sont les phytostérols et les vitamines C et E.

L'huile essentielle de *Schizandra* est riche en dérivés sesquiterpéniques tels que : d-cadinène (25,6 %), g-cadinène, b-himachalène, santalol. L'huile essentielle du fruit de *S. chinensis* contient davantage d'hydrocarbures monoterpéniques que celle de *S. sphenanthera* [Huyke et al., 2007].

Le fruit de *S. sphenanthera* se caractérise par sa richesse en désoxyschisandrine. Par contre, ses teneurs en schisandrine et g-schisandrine sont très faibles comparées à celles de la graine de *S. chinensis* (Zhu et al., 2007).

### Les extraits de fruit : formes utilisées

La forme la plus utilisée en médecine traditionnelle est le fruit séché. A côté de son emploi traditionnel en Chine à l'état déshydraté, le fruit de *Schizandra* est aussi utilisé sous forme d'extraits obtenus par des solvants d'extraction permettant d'entraîner les néo-lignanes du fruit. Les solvants mentionnés dans la littérature sont l'éthanol, le CO₂-supercritique ou CO₂-SC (associé ou pas avec un co-solvant) et l'hexane.

Plusieurs études se sont attachées à comparer les pouvoirs extractifs du CO₂-SC, du chloroforme, du méthanol et de l'éthanol à l'égard des néo-lignanes du fruit.

Selon une publication récente, l'extraction du fruit de *Schizandra* par le CO₂ ou le CO₂ + 5 % d'éthanol, ou encore par l'hexane, aboutit à des compositions d'extraits assez similaires au plan néo-lignanes. Par contre, l'utilisation d'éthanol a conduit à une moindre extraction de 2 néo-lignanes, la désydroschisandrine et la gomisine O, et apparemment une meilleure extraction de g-schisandrine (Huyke et al., 2007).

Par ailleurs, une technique d'extraction a été mise au point par Zhu et al. qui a permis de comparer les quantités de lignanes contenues respectivement dans les extraits de *Schizandra chinensis* et *Schizandra sphenanthera* (Zhu et al., 2007).

### Art antérieur

De nombreuses propriétés pharmacologiques des extraits de fruits de *Schizandra* ont été rapportées dans la littérature, telles que :
- des effets de protection hépatique, connus depuis les années 1980 ;
- des effets anti-HIV ;
- une activité anti-inflammatoire et anti-tumorale ;
- une augmentation de la biodisponibilité de certains produits, lorsque ingérés en même temps.

Ainsi, de nombreux documents citent des compositions pharmacologiques qui comprennent, entre autres composés actifs, des extraits de *Schizandra :*
La demande WO 2007/020382 de la société Phynova décrit une composition comprenant des extraits de quatre plantes dont *Schizandra chinensis* ou *Schizandra sphenanthera,* pour le traitement de problèmes hépatiques, métaboliques et/ou immunitaires, et plus particulièrement destinée à traiter l'hépatite C.
La demande WO 2007/005760 décrit une composition comprenant des composés de la famille des schizandrines, gomisines, et autres composés issus d'extraits des fruits de *Schizandra chinensis* et *Schizandra sphenanthera,* pour traiter des cellules de cancer chimiorésistantes.

L'article Huyke et al. (2007) décrit et compare les effets d'extraits de *S. sphenanthera* et *S. chinensis* sur des cellules en culture : la prolifération des cellules épidermiques de types HaCaT et A431 est inhibée de manière dose-dépendante par ces extraits, les extraits non polaires étant plus efficaces que les extraits polaires. Les auteurs de l'essai concluent que l'extrait au CO₂-SC de *Schizandra sphenanthera* pourrait être utile dans la prévention et le traitement des maladies inflammatoires et hyperprolifératives de la peau.

Aucun de ces documents ne décrit un extrait particulier de *Schizandra,* et en particulier un extrait peptidique et osidique, pour son activité en tant qu'agent neurosensoriel.

### Système neurosensoriel et peau

La peau est un organe dont l'innervation est très dense et va jusqu'aux couches les plus superficielles, à l'exception de la couche cornée. L'épiderme est également caractérisé par de nombreuses terminaisons nerveuses libres jusqu'aux couches les plus externes. Ces terminaisons nerveuses proviennent des neurones sensitifs issus des ganglions rachidiens situés le long de la moelle épinière. Ces neurones possèdent donc des axones extrêmement longs (parfois plus d'un mètre) qui transmettent les informations sensitives (tactile, chaleur..., mais également douleur) de la peau jusqu'au cerveau. La densité d'innervation varie en fonction de la zone du corps. Ainsi, chez les humains, les mains et le visage sont richement innervés par rapport au dos ou à l'abdomen.

On trouve dans la peau des fibres nerveuses sensitives et des fibres nerveuses autonomes, toutes deux appartenant au système nerveux périphérique (SNP). Les terminaisons nerveuses de type « sensitives » répondent à une variété de stimuli physiologiques tels que la chaleur, le froid, le touché, une distension mécanique et les UV. Certaines fibres peuvent même être activées par une variété de substances chimiques ou encore par des facteurs biologiques comme des agents microbiologiques ou des protéases provenant des plantes.

Les terminaisons nerveuses autonomes sont responsables des fonctions automatiques telles que la régulation de la fonction des glandes sudoripares, la vasomotricité, le flux sanguin, et, par cela, dans la thermorégulation. Ces fibres ont donc un rôle dans l'homéostasie (maintien de l'équilibre intérieur) et sont également impliquées dans des interactions complexes entre des aspects physiologiques et comportementaux

Les connexions entre nerfs et peau se font au niveau cellulaire par l'intermédiaire des neuromédiateurs (ou neurotransmetteurs). Parmi ces neuromédiateurs, on peut citer la substance P, le CGRP (Peptide Relié au Gène Calcitonine) ou encore la pro-opiomélanocortine (POMC). Ces molécules, synthétisées par les terminaisons nerveuses mais aussi par les cellules cutanées ou immunitaires, sont les médiateurs de l'échange d'information entre la peau et le système nerveux. Ces cellules expriment de nombreux récepteurs pour ces neuromédiateurs mais aussi des enzymes pouvant les dégrader (cytokines, facteurs de croissance...). Les neuromédiateurs sont ainsi capables, après liaison sur des récepteurs spécifiques, d'activer des cellules cutanées cibles comme les mastocytes, les kératinocytes, les cellules de Langerhans, les mélanocytes, les cellules de Merkel, les cellules endothéliales et les fibroblastes. D'autre part, les fonctions métaboliques des neurones cutanés peuvent être régulées par des médiateurs produits par les cellules cutanées. Cette communication cellulaire qui s'effectue dans les deux sens participe à de nombreux processus biologiques tels que l'inflammation, la réponse immunitaire, la cicatrisation, la pigmentation et la croissance pilaire. Les intrications anatomiques et fonctionnelles extrêmement étroites entre la peau et le système nerveux conduisent à parler d'un système unifié : le système neurosensoriel cutané.

Le CGRP est un peptide de 37 acides aminés, produit par les fibres nerveuses sensitives non myélinisées (type C), les cellules de Merkel et les fibres nerveuses autonomes. Le CGRP est capable d'induire des effets inflammatoires, vasodilatateurs et neurotrophiques.

Différentes protéines « sensitives » (« sensor ») sont présentes sur les neurones du système nerveux périphérique, qui sont considérés comme les seuls transducteurs de la perception cutanée. Les cellules épidermiques relaient cette transduction de signal car elles expriment également de nombreuses protéines sensitives. Ces protéines sont principalement transmembranaires et permettent la transformation de stimuli comme le toucher, la pression osmotique, la température ou des stimulations chimiques en messages biochimiques intercellulaires. Parmi ces protéines qui sont souvent des récepteurs, on peut citer la famille des TRPs (transient receptor potentials) dont les récepteurs thermo-sensibles tels que le TRPV1 (transient receptor potential vanilloide 1).

Il existe donc un dialogue entre les cellules de la peau et les neurones sensitifs. Ces interactions participent à la sensation de bien-être, un équilibre homéostatique de l'épiderme, une augmentation de la mélanogénèse.

Cependant, la situation anatomique de la peau suggère que le système nerveux est hautement exposé aux challenges environnementaux. L'homéostasie du système nerveux cutané peut être modifiée dans certaines conditions : le vieillissement, le stress, les UV, les pathologies inflammatoires, les désordres cosmétologiques... Il est donc important de protéger ce système nerveux périphérique ; cette « neuroprotection » est d'autant plus importante que le potentiel de régénération du tissu nerveux est très bas ; expliquant la récupération partielle des fonctions sensitives après blessure par exemple. Au cours du vieillissement, cette « neuroprotection » est également nécessaire pour combattre la perte de neurones associée à l'âge.

La préservation du lien anatomique entre le système nerveux central et les neurones sensitifs cutanés périphériques permet la préservation d'une bonne sensibilité, mais également un maintien des systèmes de thermorégulation et de sécrétion sébacée.

### RÉSUMÉ DE L'INVENTION

Les extraits peptidiques et osidiques de fruit de Schizandra sont connus pour leur utilisation dans la prévention ou le traitement des réactions ou pathologies de la peau. En particulier, les extraits peptidiques et osidiques de Schizandra ont une activité dans le traitement de l'acné (WO 2011012615 et WO 2011/012612).

De manière inattendue, les inventeurs ont découvert que les extraits peptidiques et osidiques de fruit de Schizandra présentent des propriétés neurosensorielles cutanées jamais décrites jusqu'à présent.

La présente invention a donc pour objet l'utilisation, notamment l'utilisation cosmétique, d'un extrait peptidique et osidique de fruit de Schizandra en tant qu'agent neurosensoriel cutané, avantageusement pour améliorer la réponse du système neurosensoriel cutané.

La présente invention a également pour objet l'utilisation d'une composition cosmétique comprenant un extrait peptidique et osidique de fruit de Schizandra, en tant qu'agent neurosensoriel, avantageusement pour améliorer la réponse du système neurosensoriel cutané.

### DESCRIPTION DES FIGURES

**Figure 1****.** Activité d'un extrait peptidique et osidique de Schizandra Sphenanthera sur l'expression génique de TRPV1 dans des kératinocytes. (* *p*<*0,05 vs cellules contrôles* - *ANOVA à un facteur suivie d'un test de Dunnett*)
**Figure 2****.** Etude de l'effet d'un extrait peptidique et osidique de Schizandra Sphenanthera sur la mobilisation de calcium intra-cytoplasmique de neurones sensitifs stimulés par la capsaïcine en co-culture avec des kératinocytes : Evolution de la fluorescence au cours du temps
**Figure 3****.** Etude de l'effet d'un extrait peptidique et osidique de Schizandra Sphenanthera sur la mobilisation de calcium intra-cytoplasmique de neurones sensitifs stimulés par la capsaïcine en co-culture avec des kératinocytes : Variation de fluorescence en pourcentage par rapport au contrôle. (# *p*<*0,05 vs contrôle* ; **p*<*0,05* *** *p*<*0,001 vs capsaicin ; Test de Bartlett's)*
**Figure 4****.** Etude de l'activité d'un extrait peptidique et osidique de Schizandra Sphenanthera sur la libération de CGRP par des neurones sensitifs stimulés par la capsaïcine en co-culture avec des kératinocytes. *(*# *p*<*0,05 vs contrôle ; ***p*<*0,001 vs capsaicine ; ANOVA à un facteur suivie d'un test de Dunnett*)
**Figure 5****.** Etude de l'effet trophique d'un extrait peptidique et osidique de Schizandra Sphenanthera sur le développement des prolongements des neurones sensitifs : longueur des neurites pour les neurones sensitifs seuls après 5 jours de culture
**Figure 6****.** Etude de l'effet trophique d'un extrait peptidique et osidique de Schizandra Sphenanthera sur le développement des prolongements des neurones sensitifs en présence de kératinocytes : longueur des neurites pour les neurones sensitifs en co-culture avec des kératinocytes après 5 jours de culture

### DESCRIPTION DETAILLÉE DE L'INVENTION

La présente invention a donc pour objectif de fournir un principe actif permettant d'améliorer la réponse du système neurosensoriel cutané. Une telle amélioration permet notamment :
- d'améliorer le prolongement des neurones, permettant ainsi d'améliorer l'innervation cutanée à l'état basai ;
- d'améliorer la réponse du système neurosensoriel cutané face aux agressions de l'environnement (douleur, chaleur, froid...) ; et
- de lutter contre la perte d'innervation cutanée liée à l'âge pour prévenir la perte de sensibilité cutanée et lutter contre l'amincissement de l'épiderme.

La présente invention a pour objet l'utilisation d'un extrait peptidique et osidique de fruit de Schizandra en tant qu'agent neurosensoriel cutané, notamment pour améliorer la réponse du système neurosensoriel cutané.

Au sens de la présente invention, on entend par « agent neurosensoriel », un composé capable d'agir sur les composants du système neurosensoriel cutané (terminsaisons nerveuses, cellules épidermiques, neuromédiateurs, récepteurs « sensitifs ») pour améliorer la réponse du système neurosensoriel cutané.

Au sens de la présente invention, on entend par « amélioration de la réponse du système neurosensoriel cutané », la préservation du lien anatomique entre le système nerveux central et les neurones sensitifs cutanés périphériques. Ceci permet une meilleure libération des informations nerveuses sous forme de neurotransmetteurs au niveau des kératinocytes et des mélanocytes et ainsi à une meilleure interaction entre les cellules de la peau et les neurones sensitifs. Ces interactions participent à la sensation de bien-être, à l'équilibre homéostatique de l'épiderme, à l'augmentation de la mélanogénèse et à la préservation d'une bonne sensibilité, au maintien des systèmes de thermorégulation et de sécrétion sébacée

Selon un premier aspect de l'invention, la présente invention a pour objet l'utilisation d'un extrait peptidique et osidique de fruit de Schizandra en tant qu'agent neurosensoriel, pour améliorer l'innervation cutanée à l'état basal, plus précisément pour améliorer la densité d'innervation cutanée en induisant un effet trophique sur les neurones conduisant à une élongation des prolongements neuronaux, plus particulièrement pour améliorer la cicatrisation et/ou pour améliorer la perception du toucher et/ou pour favoriser l'homéostasie et donc le confort cutané.

Avantageusement, ledit extrait peptidique et osidique de fruit de Schizandra peut être utilisé en tant que produit de massage. Ledit extrait peut alors être incorporé dans un produit de massage, en particulier dans un produit de massage pour bébé. De manière avantageuse, le produit de massage comprend ledit extrait peptidique et osidique de fruit de Schizandra et un excipient de massage cosmétiquement acceptable. Plus particulièrement, le produit de massage est une huile de massage, notamment une huile de massage pour bébé.

Au sens de la présente invention, on entend par « cicatrisation », la cicatrisation épidermique, ou réépithélisation, c'est-à-dire la cicatrisation des couches superficielles de l'épiderme. Le terme « cicatrisation » a donc un sens cosmétique et correspond avantageusement à la réparation de la surface de la peau et/ou à la restructuration de l'épiderme.

L'absence d'innervation sensorielle cutanée affecte négativement toutes les étapes de la cicatrisation ; en particulier un défaut d'innervation peut diminuer la prolifération kératinocytaire et induire un amincissement de l'épiderme. Il a été démontré que les neurones sensitifs influencent directement la réépithélisation (étape clé de la cicatrisation épidermique). Les neurones permettent donc une accélération de la cicatrisation, sans inflammation.

Par ailleurs l'innervation cutanée joue un rôle important dans la perception du toucher. Par l'expression « perception du toucher », on entend, au sens de la présente invention, la perception d'un contact léger avec la peau, glabre (sans poils) ou velue. Les récepteurs responsables de la perception du toucher sont en particulier les corpuscules de Meissner localisés dans la peau glabre et situés immédiatement sous l'épiderme dans les papilles dermiques et les récepteurs des follicules pileux qui sont constitués de terminaisons libres qui se situent autour de la racine du poil et sous les glandes sébacées.

Selon un second aspect de l'invention, la présente invention a pour objet l'utilisation d'un extrait peptidique et osidique de fruit de Schizandra pour lutter contre la perte d'innervation cutanée à l'état basal liée à l'âge, en particulier pour prévenir la perte de sensibilité cutanée et/ou lutter contre l'amincissement de l'épiderme.

En effet, le potentiel de régénération du tissu nerveux est très bas. La densité de l'innervation de l'épiderme décroît donc progressivement avec l'âge, entrainant ainsi une diminution de l'épaisseur épidermique et un défaut de sensation thermique dû à une densité nerveuse réduite.

Par l'expression « sensibilité cutanée », on entend, au sens de la présente invention, la sensibilité nerveuse dépendante de récepteurs situés dans la peau et stimulés par des agents extérieurs à l'organisme. Au sein de la sensibilité cutanée, on distingue : la sensibilité mécanique (ou perception du toucher), la sensibilité thermique et la sensibilité douloureuse (ou nociception).

Selon un autre aspect, la présente invention a également pour objet l'utilisation d'un extrait peptidique et osidique de fruit de Schizandra pour améliorer la perception cutanée de l'environnement, notamment pour éviter une trop grande réactivité cutanée et/ou pour lutter contre les agressions cutanées et/ou le stress cutané de l'environnement, plus particulièrement pour lutter contre les brûlures, le froid extrême et/ou la douleur.

La situation anatomique de la peau suggère que le système nerveux est hautement exposé aux challenges environnementaux, il est donc important de protéger ce système nerveux périphérique.

Au sens de la présente invention, l'expression « perception cutanée de l'environnement » signifie la perception de tout stimulus issu d'un contact direct avec l'environnement extérieur. En particulier, ces stimuli sont physiques, c'est-à-dire thermique, mécanique, électrique ou rayonnement UV, chimiques ou indirects, tels que ceux produits par des allergènes, des haptènes, des agents microbiologiques, des chocs ou une inflammation.

Au sens de la présente invention, on entend par « brûlure », toute brûlure pouvant être causée par le contact avec une source chaude, le contact avec une substance dite caustique, un frottement, l'effet de la combustion, l'effet d'un rayonnement (le coup de soleil - rayonnement ultraviolet B - rayonnement infrarouge), l'effet d'un courant électrique (électrisation) ou le froid (gelure).

Au sens de la présente invention, on entend par « froid extrême », une température située en dessous du seuil de 0 °C, avantageusement en dessous de -5 °C, plus particulièrement en dessous de -10 °C.

Avantageusement, la présente invention concerne l'utilisation d'un extrait peptidique et osidique de fruit de Schizandra pour éviter une trop grande réactivité cutanée face à l'environnement en améliorant la thermosensibilité cutanée et/ou la perception de la douleur, et/ou pour lutter contre les démangeaisons et/ou les sensations d'inconfort.

Par l'expression « thermosensibilité cutanée », on entend, au sens de la présente invention, la perception par la peau des sensations de brûlures ou de froid intenses, pouvant conduire à des réactions d'irritation cutanée.

Par l'expression « perception de la douleur », on entend, au sens de la présente invention, l'ensemble des phénomènes permettant l'intégration au niveau du système nerveux central d'un stimulus douloureux via l'activation des nocicepteurs (récepteurs à la douleur) cutanés, et pouvant conduire à des réactions d'irritation cutanée.

De manière avantageuse, la présente invention concerne l'utilisation d'un extrait peptidique et osidique de fruit de Schizandra pour prévenir ou traiter les peaux intolérantes, c'est-à-dire notamment pour moduler la réponse des peaux intolérantes face aux agressions de l'environnement.

Au sens de la présente invention, en entend par « peau intolérante », les peaux ayant un seuil de tolérance face aux agressions de l'environnement plus bas que le seuil de tolérance des peaux normales. Les peaux intolérantes réagissent de façon anormale en réponse à des stimuli extérieurs normaux et généralement bien tolérés par une peau dite normale et/ou réagissent beaucoup plus rapidement face aux agressions de l'environnement qu'une peau dite normale. Une peau dite normale est une peau sans imperfection apparente qui réagit de façon contrôlée à des agressions extérieures et dont la physiologie et la structure est considérée comme normale. Les agressions de l'environnement peuvent être physiques, c'est-à-dire thermique, mécanique, électrique ou rayonnement UV, chimiques ou indirectes, tels que ceux produits par des allergènes, des haptènes, des agents microbiologiques, des chocs ou une inflammation.

Cet abaissement du seuil de tolérance peut être la conséquence d'une hyper-réactivité cellulaire impliquant les cellules épidermiques, immunes et nerveuses.

Les réactions développées par les peaux intolérantes face à ces agressions de l'environnement peuvent être des flushs, rougeurs (érythèmes), papules, pustules, télangiectasies ; sensations de brûlure, picotements, démangeaisons, plaques rouges, sécheresse cutanée...

Selon un autre aspect, la présente invention a pour objet un extrait peptidique et osidique de fruit de Schizandra, en tant qu'agent neurosensoriel, ou une composition comprenant un tel extrait, pour son utilisation pour améliorer la cicatrisation, pour lutter contre les démangeaisons et/ou pour prévenir ou traiter les peaux intolérantes.

Dans le cadre de la présente invention, l'ensemble des modes de réalisation décrit ci-dessus peuvent être indifféremment utilisés de manière séparée ou combinée.

Avantageusement, selon la présente invention, l'extrait peptidique et osidique de fruit de Schizandra est issu de Schizandra sphenanthera.

Dans un mode préféré de l'invention, l'extrait peptidique et osidique de fruit de Schizandra est constitué de :
- 5 à 90% de peptides, et
- 5 à 90% de sucres totaux,
les pourcentages étant exprimés par rapport au poids total de la matière sèche dudit extrait peptidique et osidique.

L'extrait peptidique et osidique selon l'invention est plus avantageusement constitué de :
- 10 à 50% de peptides ;
- 10 à 60% de sucres totaux.

L'extrait peptidique et osidique selon l'invention est plus avantageusement constitué de :
- 20 à 50% de peptides ;
- 30 à 60% de sucres totaux.

Dans la présente demande, les termes « extrait peptidique et osidique de fruit de Schizandra », « extrait peptidique et osidique de Schizandra » et « extrait de Schizandra » ont la même signification et sont utilisés indifféremment l'un de l'autre pour désigner le même extrait de fruit.

En particulier l'extrait peptidique et osidique de fruit de Schizandra ne comprend pas de lignanes, connus jusqu'à présent pour être les principes actifs du fruit.

Selon un mode préférentiel de réalisation de l'invention, l'extrait peptidique et osidique de fruit de Schizandra est avantageusement obtenu par un procédé comprenant les étapes successives suivantes : à partir de fruit de *Schizandra,* une extraction par CO₂ supercritique permet d'obtenir une huile brute et un tourteau délipidé. Le tourteau de baies de *Schizandra,* obtenu après extraction des lipides, est dispersé dans l'eau. Il est ensuite réalisé une hydrolyse de l'amidon et des fibres (cellulose, hémicellulose, ...) à l'aide d'un mélange de cellulases et d'alpha-amylases, ainsi que des protéines par des protéases. Un traitement thermique permet de dénaturer les enzymes en fin de réaction. Après centrifugation, le milieu réactionnel est purifié en réalisant une ultrafiltration et une diafiltration sur une membrane ayant un seuil de coupure de 15 kDa afin d'éliminer les protéines résiduelles (retentât). Le perméat est ensuite nanofiltré afin d'éliminer des sels minéraux, puis l'extrait peptidique et osidique est décoloré avec du charbon actif puis filtré et récupéré. Enfin, le produit est filtré stérilement (0,2 µm) et peut être lyophilisé ou conditionné. De manière avantageuse, le produit est lyophilisé en présence de maltodextrine.

**Tableau 1 : Exemple d'une composition analytique d'un extrait peptidique et osidique de Schizandra, en pourcentages par rapport à la matière sèche :**

| | |
|---|---|
| Teneur en peptides | 13% |
| Teneur en azote alpha aminé | 6.0% |
| Teneur en sucres totaux | 44% |

| Répartition des masses molaires (en Daltons) des peptides | |
|---|---|
| > 3500 Da | 0% |
| 3500 - 1200 Da | 4% |
| 1200 - 300 Da | 23% |
| 300 - 130 Da | 18% |
| < 130 Da | 55% |

L'extrait peptidique et osidique de fruit de Schizandra peut être formulé sous forme d'une composition cosmétique. Ainsi, la présente invention porte également sur l'utilisation d'une composition cosmétique comprenant un extrait peptidique et osidique de fruit de Schizandra, en tant qu'agent neurosensoriel, avantageusement pour améliorer la réponse du système neurosensoriel cutané.

Avantageusement, la composition cosmétique comprenant un extrait peptidique et osidique de fruit de Schizandra, en tant qu'agent neurosensoriel, peut être utilisée pour :
- améliorer l'innervation cutanée à l'état basal,
- améliorer la cicatrisation,
- améliorer la perception du toucher, notamment comme produit de massage
- lutter contre la perte d'innervation cutanée à l'état basal liée à l'âge, avantageusement pour prévenir la perte de sensibilité cutanée et/ou lutter contre l'amincissement de l'épiderme,
- améliorer la perception cutanée de l'environnement, plus particulièrement pour lutter contre les brûlures, le froid extrême et/ou la douleur,
- améliorer la thermosensibilité cutanée et/ou la perception de la douleur,
- lutter contre les démangeaisons et/ou les sensations d'inconfort, et/ou
- prévenir ou traiter les peaux intolérantes.

La composition cosmétique peut comprendre de 0,01 à 15 %, en particulier de 0.01% à 5%, en poids dudit extrait peptidique et osidique de fruit de Schizandra, par rapport au poids total de la composition. De telles concentrations peuvent constituer des concentrations efficaces d'extrait peptidique et osidique de fruit de Schizandra.

La composition cosmétique peut par ailleurs contenir un ou plusieurs excipient(s) cosmétiquement acceptable(s), tels que des gélifiants, par exemple des gélifiants hydrophiles ou lipophiles, des conservateurs, des agents antioxydants, des solvants, des parfums, des charges, des filtres chimiques ou minéraux, des pigments, des agents chélateurs, des absorbeurs d'odeur, des eaux thermales et/ou des matières colorantes. Les quantités de ces différents excipients sont celles classiquement utilisées en cosmétique. Par exemple, la quantité de chaque excipient peut varier de 0,01% à 20% en poids, par rapport au poids total de la composition cosmétique.

La composition selon l'invention peut en outre comprendre au moins un agent anti-inflammatoire/anti-irritant, un agent antioxydant/antiradicalaire, un agent cicatrisant/réparateur de la barrière, un agent anti-âge, et/ou un agent hydratant.

Les agents anti-inflammatoires / anti-irritants limitent la réaction inflammatoire conduite via les cytokines ou médiateurs du métabolisme de l'acide arachidonique et ont des propriétés apaisantes et anti-irritantes. Les plus classiques sont l'acide glycyrrhétinique (les dérivés de réglisse) avec ses sels et esters, l'alpha bisabolol, le ginkgo biloba, le calendula, l'acide lipoïque, le beta-carotène, la vitamine B3 (niacinamide, nicotinamide), la vitamine E, la vitamine C, la vitamine B12, les flavonoïdes (thé vert, quercétine ...), le lycopène ou la lutéine, les sucres d'Avocat, l'oléodistillat d'Avocat, l'arabinogalactane, les peptides de Lupin, un extrait total de Lupin, un extrait peptidique de Quinoa, le Cyclocéramide® (dérivé d'oxazoline), les substances anti-glycation telles que la carnosine, la n-acétyl-cystéine, les isoflavones comme par exemple la génistéine/génistine, daidzéine/daidzine, les eaux de source ou thermales (eau d'Avène, eau de la Roche Posay, eau de Saint Gervais, eau d'Uriage, eau de Gamarde), les extraits de Goji (Lycium barbarum), les peptides ou complexes d'acides aminés végétaux ou encore. la disulone topique, ou les médicaments anti-inflammatoires.

Par agent antioxydant, on entend une molécule qui diminue ou empêche l'oxydation d'autres substances chimiques. Les antioxydants/antiradicalaires pouvant être utilisés en association sont avantageusement choisis dans le groupe constitué par les thiols et les phénols, par les dérivés de réglisse comme l'acide glycyrrhétinique avec ses sels et esters, l'alpha bisabolol, l'extrait de Ginkgo biloba, de Calendula, le Cyclocéramide® (dérivé d'oxazoline), les peptides d'Avocat, les oligo-éléments comme le cuivre, le zinc, et le sélénium, l'acide lipoïque, la vitamine B12, la vitamine B3 (niacinamide, nicotinamide), les vitamines C, les vitamines E, le co-enzyme Q10, le krill, le glutathion, le butylhydroxytoluène (BHT), le butylhydroxyanisol (BHA), le lycopène ou la lutéine, le béta-carotène, la famille des polyphénols comme les tanins, les acides phénoliques, les anthocyanes, les flavonoïdes avec par exemple les extraits de thé vert, de fruits rouges, de cacao, de raisin, de *Passiflora incarnata,* de Citrus ou encore les isoflavones comme par exemple la génistéine/génistine, daidzéine/daidzine, Dans le groupe des anti-oxydants on trouve aussi les substances anti-glycation telles que la carnosine ou certains peptides, la n-acétyl-cystéine, ainsi que les enzymes antioxydants ou antiradicalaires comme la SOD (super oxyde dismutase), la catalase, la glutathion peroxydase, la thioredoxine réductase et leurs agonistes.

Les agents cicatrisants et/ ou réparateurs de la fonction barrière pouvant être utilisés en association sont avantageusement la vitamine A, le panthénol (vitamine B5), l'Avocadofurane®, les sucres d'Avocat, le lupéol, l'extrait peptidique de Maca, un extrait peptidique de Quinoa, l'arabinogalactane, l'oxyde zinc, le magnésium, le silicium, l'acide madécassique ou asiatique, le sulfate de dextran, le coenzyme Q10, la glucosamine et ses dérivés, la chondroïtine sulfate et globalement les GAG (glycosaminoglycanes), le sulfate de dextran, les céramides, le cholestérol, le squalane, les phospholipides, les peptides de Soja fermentés ou non, les peptides végétaux, les polysaccharides marins, végétaux ou biotechnologiques comme les extraits d'algues ou l'extrait de fougère, les oligo-éléments, les extraits de plantes à tanins comme les tanins dérivant de l'acide gallique appelés tanins galliques ou encore hydrolysables, initialement trouvés dans la noix de galle, et les tanins catéchiques résultant de la polymérisation d'unités flavanniques dont le modèle est fourni par le Cachou (*Acacia catechu*). Les oligo-éléments pouvant être utilisés sont avantageusement choisis dans le groupe constitué par le cuivre, le magnésium, le manganèse, le chrome, le sélénium, le silicium, le zinc et leurs mélanges.

Les agents anti-âge pouvant agir en association pour la prise en charge de l'acné sur les sujets d'âge mûr sont des agents antioxydants et en particulier la vitamine C, la vitamine A, le rétinol, le rétinal, l'acide hyaluronique de tout poids moléculaire, l'Avocadofurane®, les peptides de Lupin, l'extrait peptidique de Maca.
Les actifs hydratants / émollients les plus utilisés sont la glycérine ou ses dérivés, l'urée, l'acide pyrrolidone carboxylique et ses dérivés, l'acide hyaluronique de tous poids moléculaires, les glycosaminoglycanes et tous autres polysaccharides d'origine marine, végétale ou biotechnologique comme par exemple la gomme xanthane, le fucogel®, certains acides gras comme l'acide laurique, l'acide myristique, les acides gras mono- et poly-insaturés de type oméga 3, 6 et 7, 9 (exemple : l'acide linoléique, l'acide palmitoléique, etc), l'Oléodistillat de Tournesol, les peptides d'Avocat, le beurre de Cupuaçu.

Une association particulièrement avantageuse selon l'invention est une composition comprenant l'extrait peptidique et osidique de fruit de *Schizandra sphenanthera* et des insaponifiables végétaux et animaux, comme par exemple, les insaponifiables d'Avocat et de Soja, et des concentrats d'huile végétale ou animale en insaponifiables, comme par exemple le concentrat d'huile de Tournesol ou d'huile de Palme, ou encore des huiles végétales contenant des insaponifiables comme par exemple les huiles de Soja et de Colza, et les dérivés d'insaponifiables comme les furanes d'Avocat, les esters de stérols et les dérivés vitaminiques.

Une association particulièrement avantageuse selon l'invention est une composition comprenant l'extrait peptidique et osidique de fruit de *Schizandra sphenanthera* et des sucres d'Avocat (voir demande WO 2005/115421). Cette composition est particulièrement adaptée pour le traitement de la réparation de la barrière cutanée et de l'inflammation.

Une association particulièrement avantageuse selon l'invention est une composition comprenant l'extrait de fruit peptidique et osidique de *Schizandra sphenanthera* et des peptides d'Avocat (voir demande internationale WO 2005/105123). Cette composition est particulièrement adaptée pour le traitement de l'irritation et de l'inflammation.

Une autre association particulièrement avantageuse selon l'invention est une composition comprenant l'extrait peptidique et osidique de fruit de *Schizandra sphenanthera* et une huile d'Avocat (voir les demandes internationales WO 2004/012496, WO 2004/012752, WO 2004/016106, WO 2007/057439).

Une autre association particulièrement avantageuse selon l'invention est une composition comprenant l'extrait peptidique et osidique de fruit de *Schizandra sphenanthera* et l'Avocadofurane® (furanes d'Avocat, pouvant être obtenus par le procédé décrit dans la demande internationale WO 01/21605). Cette composition est particulièrement adaptée pour le traitement de l'inflammation, pour favoriser la cicatrisation, et pour ses propriétés anti-âge.

Une autre association particulièrement avantageuse selon l'invention est une composition comprenant l'extrait peptidique et osidique de fruit de *Schizandra sphenanthera* et les insaponifiables d'Avocat et de Soja pouvant être utilisés en association sont avantageusement un mélange d'insaponifiables d'avocat furanique et d'insaponifiables de Soja, dans un rapport respectif d'environ 1/3-2/3. Les insaponifiables d'Avocat et de Soja sont encore plus avantageusement le produit Piasclédine®, commercialisé par les Laboratoires Expanscience.

Une autre association particulièrement avantageuse selon l'invention est une composition comprenant l'extrait peptidique et osidique de fruit de *Schizandra sphenanthera* et un Oléodistillat de Tournesol, encore plus avantageusement avec un Oléodistillat de Tournesol comportant majoritairement de l'acide linoléique, tel que l'actif commercialisé par les Laboratoires Expanscience, Soline® (cf. la demande internationale WO 01/21150), Cette composition est particulièrement adaptée pour le traitement de l'inflammation et pour la réparation de la barrière cutanée.

Une autre association particulièrement avantageuse selon l'invention est une composition comprenant l'extrait peptidique et osidique de fruit de *Schizandra sphenanthera* et un insaponifiable de Soja, tel qu'obtenu selon le procédé décrit dans la demande internationale WO 01/51596.

Une autre association particulièrement avantageuse selon l'invention est une composition comprenant l'extrait peptidique et osidique de fruit de *Schizandra sphenanthera* et du Lupéol (FR 2 822 821, FR 2 857 596). Cette composition est particulièrement adaptée pour favoriser la cicatrisation.

Une autre association particulièrement avantageuse selon l'invention est une composition comprenant l'extrait peptidique et osidique de fruit de *Schizandra sphenanthera* et des peptides de Lupin tels qu'obtenus selon le procédé décrit dans la demande WO 2005/102259. Cette composition est particulièrement adaptée pour le traitement de l'inflammation et est utilisée pour ses propriétés anti-âge.

Une autre association particulièrement avantageuse selon l'invention est une composition comprenant l'extrait peptidique et osidique de fruit de *Schizandra sphenanthera* et un extrait total de Lupin (voir demande internationale WO 2005/102259). Cette composition est particulièrement adaptée pour le traitement des irritations.

Une autre association particulièrement avantageuse selon l'invention est une composition comprenant l'extrait peptidique et osidique de fruit de *Schizandra sphenanthera* et une huile de Lupin, avantageusement une huile de Lupin blanc doux, telle que celle décrite dans la demande internationale WO 98/47479.

Une autre association particulièrement avantageuse selon l'invention est une composition comprenant l'extrait peptidique et osidique de fruit de *Schizandra sphenanthera* et un extrait peptidique de Maca (voir demande internationale WO 2004/112742). Cette composition est particulièrement appréciée pour ses propriétés cicatrisante et anti-âge.

Une association particulièrement avantageuse selon l'invention est une composition comprenant l'extrait de fruit peptidique et osidique de *Schizandra sphenanthera* et des peptides de riz (voir demande internationale WO 2008/009709). Cette composition est particulièrement appréciée pour ses propriétés de stimulation de la mélanogénèse et du transfert de la mélanine.

Une autre association particulièrement avantageuse selon l'invention est une composition comprenant l'extrait peptidique et osidique de fruit de *Schizandra sphenanthera* et du Cyclocéramide® (dérivé d'oxazoline) tel que décrit dans les demandes internationales WO 2004050052, WO 2004050079 et WO 2004112741. Cette composition est particulièrement adaptée pour le traitement des réactions inflammatoires.

Une autre association particulièrement avantageuse selon l'invention est une composition comprenant l'extrait peptidique et osidique de fruit de *Schizandra sphenanthera* et un extrait de Quinoa, en particulier un extrait peptidique (voir la demande internationale WO 2008/080974). Cette composition est particulièrement adaptée pour le traitement des conditions inflammatoires et de la réparation de la barrière cutanée.

Une autre association particulièrement avantageuse selon l'invention est une composition comprenant l'extrait peptidique et osidique de fruit de *Schizandra sphenanthera* et de beurre de Cupuaçu. Cette composition est particulièrement appréciée pour ses propriétés hydratantes.

Une autre association particulièrement avantageuse selon l'invention est une composition comprenant l'extrait peptidique et osidique de fruit de *Schizandra sphenanthera* et un Oléodistillat de Colza.

Une autre association avantageuse selon l'invention est une composition comprenant l'extrait peptidique et osidique de fruit de *Schizandra sphenanthera* et un Oléodistillat de Maïs.

Toutes ces associations comprennent au moins un autre composé actif, en plus de l'extrait de fruit de *Schizandra sphenanthera,* et peuvent comprendre deux, trois, quatre ou plus de composés actifs tels que décrits précédemment.

Outre ces actifs, l'extrait peptidique et osidique de fruit de *Schizandra sphenanthera* selon l'invention, seul ou en association avec les actifs précédemment cités, peut être utilisé en association des actifs protecteurs solaires, tels que des filtres ou écrans solaires UVB et/ou UVA ; tels les écrans ou filtres minéraux et/ou organiques connus de l'homme du métier qui adaptera leur choix et leurs concentrations en fonction du degré de protection recherché.

A titre d'exemple d'actifs protecteur solaire, on peut notamment citer le dioxyde de titane, l'oxyde de zinc, le méthylène bis-benzotriazolyl tétraméthylbutylphénol (nom commercial : TINOSORB M) et le Bis-éthylhéxyloxyphénol méthoxyphényl triazine (nom commercial : TINOSORB S), l'octocrylène, le butyl méthoxydibenzoylméthane, l'acide terephthalylidene dicamphor sulfonique, le 4-méthylbenzylidène de camphre, la benzophénone, l'éthylhexyl méthoxycinnamate, l'éthylhexyl diméthyl PABA, le diéthylhexyl butamido triazone.

La composition selon l'invention peut être formulée sous la forme de différentes préparations adaptées à une administration topique, telles que les crèmes, les gels, les émulsions, les laits, les pommades, les lotions, les huiles, les solutions aqueuses ou hydro-alcooliques ou glycoliques, les poudres, les patchs, les sprays ou tout autre produit pour application externe. De telles formulations sont présentées dans les exemples ci-après.

Les exemples qui suivent visent à illustrer la présente invention.

### EXEMPLES

### Exemple 1 : Effet d'un extrait peptidique et osidique de Schizandra Sphenanthera sur l'expression génique de TRPV1

Il est possible, en culture, de modéliser le mécanisme de potentiels d'action antidromiques. Les neurones sensitifs, issus des ganglions rachidiens, sont cultivés en présence de kératinocytes. Après 10 jours, les neurones sont matures, ils libèrent spontanément des neuropeptides tels que le CGRP et la substance P au niveau des kératinocytes. Cette libération est dite basale, elle peut être mesurée par des kits de dosage ELISA.

Le récepteur TRPV1 (Transient Receptor Potential Vanilloid 1) est une protéine membranaire qui appartient à la famille des canaux cationiques non sélectifs des TRP. C'est un nocicepteur répondant, entre autres, à une activation par la capsaïcine.

Au niveau de la peau, TRPV1 est exprimé par les kératinocytes, les mastocytes et les fibres nerveuses. En réponse à une stimulation thermique (> 43°C), chimique ou mécanique, l'activation de TRPV1 conduit au relargage de cytokines et de neuropeptides conduisant à une réponse neurosensorielle (activation des nerfs sensitifs) qui se traduit par des sensations de douleur, prurit, inconfort, flush.

L'activation de TRPV1 induit également une activation du système vasculaire, de façon directe ou indirecte, qui peut notamment aboutir à un effet vasodilatateur puissant.

### Matériel et méthodes

Des kératinocytes épidermiques humains normaux ont été incubés pendant 48 heures en présence de l'extrait peptidique et osidique de Schizandra Sphenanthera à 0,05% (MS).

A la fin du traitement, les surnageants de culture ont été éliminés et les ARN totaux extraits à l'aide du kit d'extraction RNeasy MiniKit [Qiagen]. Les ARN totaux ont ensuite été dosés en mini chips à l'aide du système Experion™ et du kit Experion RNA StdSens [Biorad] puis réverse-transcrits en cDNA à l'aide du kit iScript cDNA Synthesis [Biorad].

Les cDNA néo-synthétisés relatifs au gène cible TRPV1 ou au gène de référence GAPDH ont été amplifiés sélectivement par PCR en temps réel sur iQ5 [Biorad] par la technologie SybrGreen [kit iQ SybrGreen, Biorad].

La significativité des résultats a été vérifiée par une analyse de variance à un facteur suivie d'un test de Dunnett (logiciel GraphPad Prism version 5.02, GraphPad Software, San Diego California USA).

### Résultats

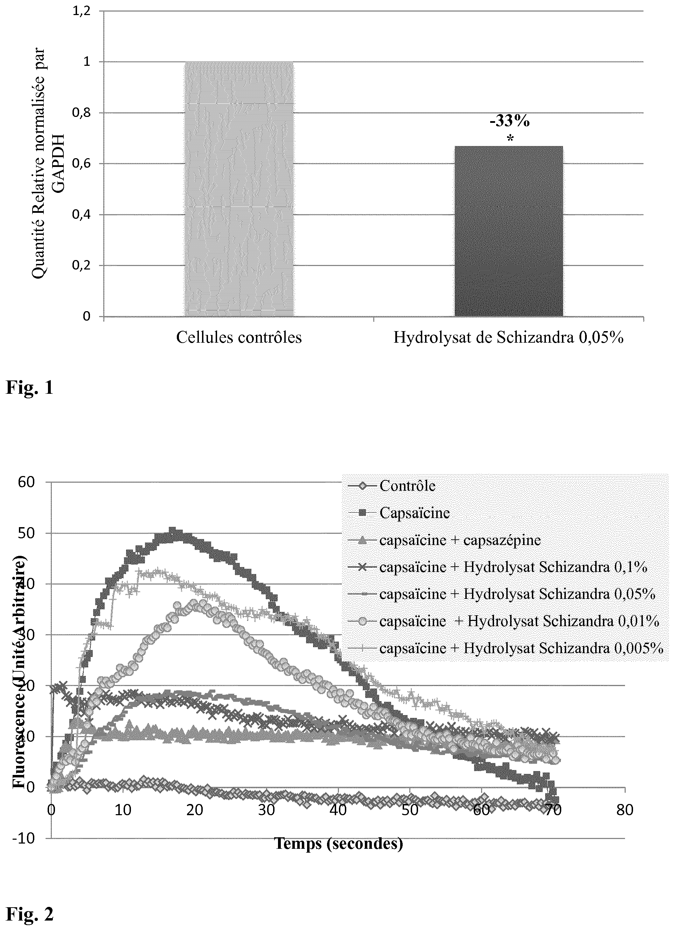

L'extrait peptidique et osidique de Schizandra Sphenanthera à 0,05% a significativement inhibé l'expression génique de TRPV1 dans des kératinocytes (-33%, p<0,05 ; Figure 1).

### Conclusion

Par son action inhibitrice sur le récepteur TRPV1, l'extrait peptidique et osidique de Schizandra Sphenanthera a une action en tant qu'agent neurosensoriel, notamment pour améliorer la perception cutanée de l'environnement, notamment pour éviter une trop grande réactivité cutanée et/ou pour lutter contre les agressions cutanées et/ou le stress cutané de l'environnement, plus particulièrement pour lutter contre les brûlures, le froid extrême et/ou la douleur.

### Exemple 2 : Effet d'un extrait peptidique et osidique de Schizandra Sphenanthera sur l'activité du récepteur TRPV1

L'activation du récepteur TRPV1 sur des neurones sensitifs peut être évaluée en analysant l'activité électrique de neurones sensitifs par suivi des variations de concentration de calcium dans le cytoplasme.

En effet, après stimulation des cellules par de la capsaïcine, le calcium extra-cellulaire entre massivement dans le cytoplasme des neurones afin de déclencher une activité électrique et provoquer une libération massive de neuropeptides dans l'espace extra-cellulaire.

La variation du niveau de fluorescence est analysée par l'incorporation d'une sonde fluorescente calcium dépendante (Fluo-4) dans les cellules. Ainsi, lors de l'augmentation du calcium dans les cellules, le niveau de fluorescence augmente dans le corps cellulaire des neurones. Plus ce niveau est important, plus la stimulation des neurones est importante.

### Matériel et méthodes

Des neurones sensitifs cultivés en présence de kératinocytes épidermiques humains normaux, ont été incubés en présence de l'extrait peptidique et osidique de Schizandra Sphenanthera à 0,005% ; 0,01% ; 0,05% et 0,1% (MS) ou de la molécule de référence capsazépine à 10⁻⁵M (Sigma).

Après 60 minutes de pré-incubation, la sonde fluorescente Fluo-4 (Molecular probe) a été ajoutée dans le milieu de culture et les cellules ont été incubées 30 minutes. Après rinçage, les co-cultures ont été observées en épi-fluorescence pendant 70 secondes ; 5 secondes après le début de l'enregistrement, les neurones (en co-culture) ont été stimulés par de la capsaïcine à 10⁻⁶M (Sigma).

La significativité des résultats a été vérifiée par un test de Bartlett's.

### Résultats

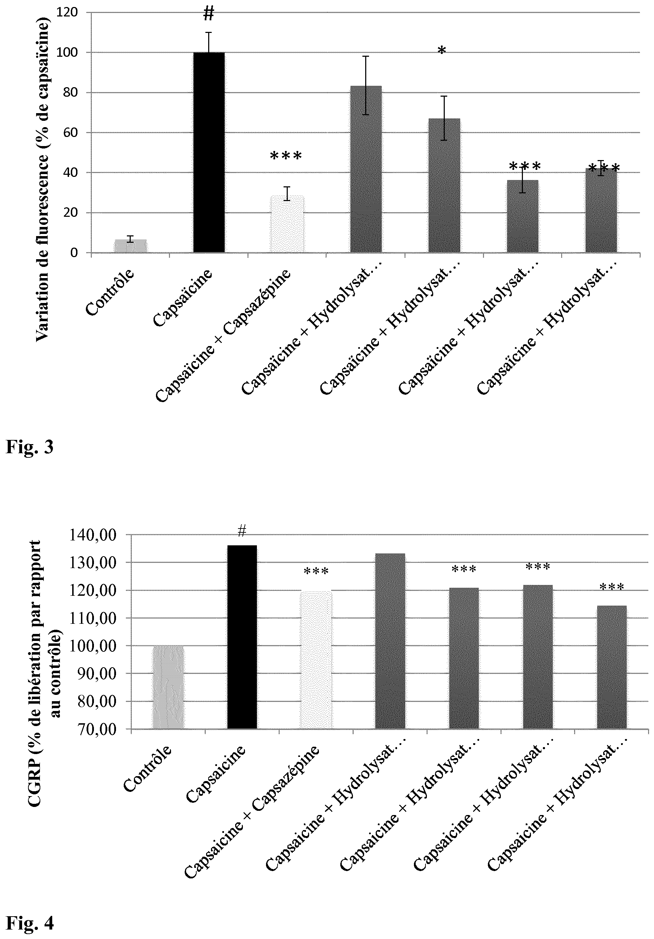

Comme le montrent les Figures 2 et 3, l'ajout de capsaïcine entraine une augmentation significative du niveau de fluorescence et donc de la quantité de calcium intra-cytoplasmique reflétant l'activation du récepteur TRPV1.

L'extrait peptidique et osidique de Schizandra Sphenanthera a significativement inhibé la mobilisation de calcium intra-cytoplasmique visualisée par une diminution de la variation de fluorescence. L'extrait peptidique et osidique de Schizandra Sphenanthera est donc capable d'inhiber l'activité du récepteur TRPV1.

L'extrait peptidique et osidique de Schizandra Sphenanthera a donc bien une action en tant qu'agent neurosensoriel, notamment pour améliorer la perception cutanée de l'environnement, notamment pour éviter une trop grande réactivité cutanée et/ou pour lutter contre les agressions cutanées et/ou le stress cutané de l'environnement, plus particulièrement pour lutter contre les brûlures, le froid extrême et/ou la douleur.

### Exemple 3 : Etude de l'activité d'un extrait peptidique et osidique de Schizandra Sphenanthera sur la libération de CGRP par des neurones sensitifs stimulés par la capsaïcine en co-culture avec des kératinocytes

La capsaïcine, irritant naturel de la famille des vanilloïdes, est le constituant actif d'une large variété de piments. Cette toxine, agit essentiellement sur les fibres sensorielles amyéliniques C, notamment celles véhiculant les messages nociceptifs. L'effet de la capsaïcine résulte de l'activation d'un récepteur dénommé récepteur vanilloïde (TRPV1) présent préférentiellement au niveau des terminaisons nerveuses de l'épiderme. En présence de capsaïcine dans le milieu de culture, les neurones sensitifs augmentent la quantité de neuropeptides libérés. La capsazépine est un antagoniste spécifique du récepteur TRPV1.

### Matériel et méthodes

Des neurones sensitifs ont été cultivés en présence de kératinocytes épidermiques humains normaux. Après 3 jours de co-culture, les neurones émettent des prolongements sous forme de terminaisons libres en contact avec les kératinocytes.

Les cellules ont alors été incubées en présence de l'extrait peptidique et osidique de Schizandra Sphenanthera à 0,005% ; 0,01% ; 0,05% et 0,1% (MS) ou de la molécule de référence capsazépine à 10⁻⁵M (Sigma).

Après 60 minutes de pré-incubation, les neurones (en co-culture) ont été stimulés par de la capsaïcine à 10⁻⁷M (Sigma).

Après 15 minutes de stimulation, les surnageants de culture ont été récupérés afin de doser le CGRP libéré. Le dosage de CGRP a été réalisé à l'aide du kit de dosage CGRP (SpiBio).

La significativité des résultats a été vérifiée par une analyse de variance à un facteur suivie d'un test de Dunnett.

### Résultats

L'extrait peptidique et osidique de Schizandra Sphenanthera a significativement inhibé la libération de CGRP induite par la capsaïcine dans des neurones sensitifs en co-culture avec des kératinocytes (Figure 4). Cet effet inhibiteur est similaire à celui induit par la capsazépine, inhibiteur de référence.

### Conclusion

L'extrait peptidique et osidique de Schizandra Sphenanthera est capable d'inhiber le récepteur TRPV1 au niveau de l'expression génique ainsi qu'au niveau de l'activation, comme montré par l'effet inhibiteur du relargage de CGRP dans des co-cultures neurones/kératinocytes stimulées par de la capsaïcine (agoniste spécifique du récepteur TRPV1).

L'effet inhibiteur de l'extrait peptidique et osidique de Schizandra Sphenanthera vis-à-vis de l'activation du TRPV1 est comparable à celui de la capsazépine, antagoniste spécifique du TRPV1.
Par son action inhibitrice sur le récepteur TRPV1, l'extrait peptidique et osidique de Schizandra Sphenanthera contribue à moduler la réponse neurosensorielle cutanée Permettant ainsi d'améliorer la perception cutanée de l'environnement, notamment pour éviter une trop grande réactivité cutanée et/ou pour lutter contre les agressions cutanées et/ou le stress cutané de l'environnement, plus particulièrement pour lutter contre les brûlures, le froid extrême et/ou la douleur.

### Exemple 4 : Etude de l'effet trophique d'un extrait peptidique et osidique de Schizandra Sphenanthera sur le développement des prolongements des neurones sensitifs en présence de kératinocytes

La longueur des prolongements de neurones sensitifs en co-culture avec des kératinocytes a été mesurée à différents stades de développement.

### Matériel et méthodes

Des neurones sensitifs cultivés seuls ou en présence de kératinocytes épidermiques humains normaux, ont été incubés en présence de l'extrait peptidique et osidique de Schizandra Sphenanthera à 0,005% ; 0,01% ; 0,05% et 0,1% (MS) ou de la molécule de référence NGF à 20 ng/ml (Sigma).

Après 5 jours de stimulation, les cellules ont été fixées et marquées par un anticorps anti β-tubuline. L'analyse du réseau de neurites a été réalisée à l'aide du logiciel Developer (GE Healthcare).

La significativité des résultats a été vérifiée par une ANOVA à un facteur suivie d'un test de Dunnett.

### Résultats

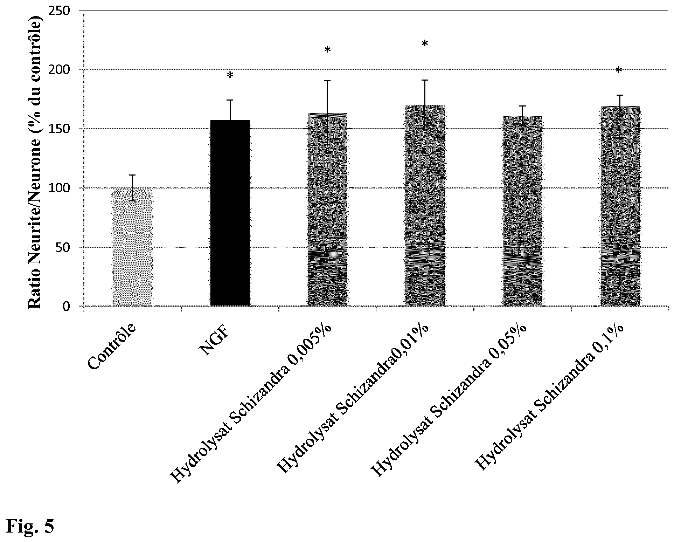

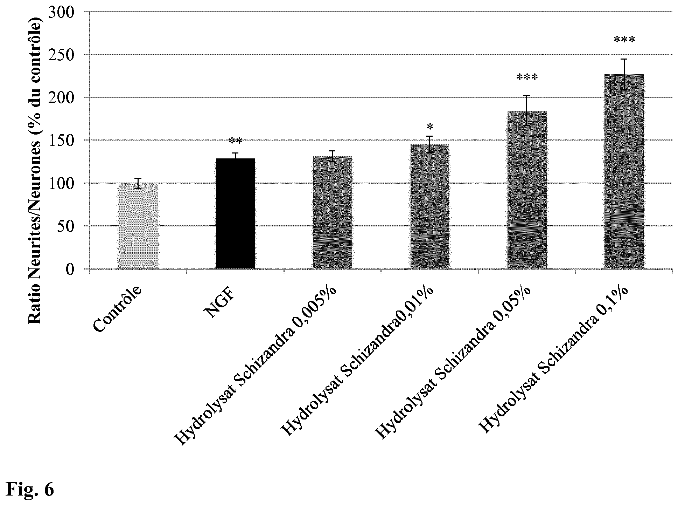

Comme le montrent les Figures 5 et 6, l'extrait peptidique et osidique de Schizandra Sphenanthera a entraîné une augmentation significative de la longueur des prolongements neuronaux. Cet effet trophique est potentialisé en présence de kératinocytes, en effet dans les conditions de co-culture, l'augmentation observée est supérieure et dose-dépendante ; l'extrait peptidique et osidique de Schizandra Sphenanthera peut donc induire une augmentation de facteurs neurotrophiques par les kératinocytes.
L'extrait peptidique et osidique de Schizandra Sphenanthera a donc une action en tant qu'agent neurosensoriel pour améliorer l'innervation cutanée à l'état basal.

### Conclusion

L'extrait peptidique et osidique de Schizandra Sphenanthera est capable d'inhiber le récepteur TRPV1 au niveau de l'expression génique ainsi qu'au niveau de l'activation, comme montré par l'effet sur la mobilisation de calcium cytoplasmique ainsi que l'effet inhibiteur du relargage de CGRP dans des co-cultures neurones/kératinocytes stimulées par de la capsaïcine (agoniste spécifique du récepteur TRPV1).

L'effet inhibiteur de l'extrait peptidique et osidique de Schizandra Sphenanthera vis-à-vis de l'activation du TRPV1 est comparable à celui de la capsazépine, antagoniste spécifique du TRPV1.

Par son action inhibitrice sur le récepteur TRPV1, l'extrait peptidique et osidique de Schizandra Sphenanthera contribue à moduler la réponse neurosensorielle cutanée.

D'autre part, par l'effet trophique positif exercé sur des neurones sensitifs, l'extrait peptidique et osidique de Schizandra Sphenanthera exerce un effet bénéfique pour la qualité de l'innervation cutanée.

### Exemple 5 : Exemples de formulations cosmétiques

### EMULSION Huile dans Eau N°1

| **Matière première / Nom commercial** | **%** |
|---|---|
| POLYDECENE HYDROGENE | De 5 à 20 % |
| LAURYLGLUCOSIDE-GLYSTEARATE | De 1 à 5 % |
| DICAPRYLYL CARBONATE | De 1 à 5 % |
| GLYCEROL | De 5 à 20 % |
| CARBOPOL | De 0 à 1 % |
| GOMME XANTHANE | De 0 à 1 % |
| **EXTRAIT DE SCHIZANDRA** | **De 0,01 à 5 %** |
| LESSIVE SOUDE | De 0 à 1 % |
| CONSERVATEURS | De 0 à 1 % |
| ACIDE CITRIQUE | De 0 à 1 % |
| EAU PURIFIÉE | QSP 100 % |

### EMULSION Huile dans Eau N°2

| **INCI UE** | **% INCI** |
|---|---|
| AQUA | QSP |
| GLYCERINE | 1 à 10% |
| PROPANEDIOL DICAPRYLATE | 1 à 10% |
| DICAPRYLYL CARBONATE | 1 à 10% |
| GLYCERYL STEARATE CITRATE | 1 à 5% |
| CETYL ALCOHOL | 1 à 5% |
| GOMME XANTHANE | 0 à 2% |
| **EXTRAIT DE SCHIZANDRA** | **De 0,01 à 5 %** |
| TOCOPHEROL | 0 à 0.5% |
| CONSERVATEURS | 0 à 2% |
| AJUSTEUR pH | 0 à 1% |
| | 100.000000 |

### EMULSION Eau dans Huile

| **Matière première / Nom commercial** | **%** |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| **EXTRAIT DE SCHIZANDRA** | **De 0,01 à 5 %** |
| ISONONYL ISONONANOATE | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

## Revendications

1. Extrait peptidique et osidique de fruit de Schizandra pour son utilisation en tant qu'agent neurosensoriel cutané.

2. Extrait pour son utilisation selon la revendication 1, pour améliorer l'innervation cutanée à l'état basal.

3. Extrait pour son utilisation selon la revendication 1 ou 2, pour améliorer la cicatrisation.

4. Extrait pour son utilisation selon la revendication 1 ou 2, pour améliorer la perception du toucher, avantageusement en tant que produit de massage.

5. Extrait pour son utilisation selon la revendication 1 ou 2, pour lutter contre la perte d'innervation cutanée à l'état basal liée à l'âge, avantageusement pour prévenir la perte de sensibilité cutanée et/ou lutter contre l'amincissement de l'épiderme.

6. Extrait pour son utilisation selon la revendication 1, pour améliorer la perception cutanée de l'environnement, notamment pour éviter une trop grande réactivité cutanée et/ou pour lutter contre les agressions cutanées et/ou le stress cutané de l'environnement, plus particulièrement pour lutter contre les brûlures, le froid extrême et/ou la douleur.

7. Extrait pour son utilisation selon la revendication 6, pour améliorer la thermosensibilité cutanée et/ou la perception de la douleur.

8. Extrait pour son utilisation selon la revendication 6, pour lutter contre les démangeaisons et/ou sensations d'inconfort.

9. Extrait pour son utilisation selon la revendication 6, pour prévenir ou traiter les peaux intolérantes.

10. Extrait pour son utilisation selon l'une quelconque des revendications précédentes, dans laquelle ledit extrait peptidique et osidique de fruit de Schizandra est constitué de :
- 10 à 50% de peptides, et
- 10 à 60 % de sucres totaux,
les pourcentages étant exprimés par rapport au poids total dudit extrait peptidique et osidique.

11. Extrait pour son utilisation selon l'une quelconque des revendications précédentes, en application topique.

## Patentansprüche

1. Peptid- und Osidextrakt aus Schisandrafrucht für seine Verwendung als neurosensorisches kutanes Mittel.

2. Extrakt für seine Verwendung nach Anspruch 1 zur Verbesserung der kutanen Innvervierung im Basalzustand.

3. Extrakt für seine Verwendung nach Anspruch 1 oder 2 zur Verbesserung der Heilung.

4. Extrakt für seine Verwendung nach Anspruch 1 oder 2 zur Verbesserung der Wahrnehmung der Berührung, in vorteilhafter Weise als Massageprodukt.

5. Extrakt für seine Verwendung nach Anspruch 1 oder 2 zur Bekämpfung des Verlusts der kutanen Innervierung im Basalzustand in Verbindung mit dem Alter, in vorteilhafter Weise zur Vorbeugung des Verlusts der kutanen Sensibilität und/oder zur Bekämpfung des Dünnerwerdens der Epidermis.

6. Extrakt für seine Verwendung nach Anspruch 1 zur Verbesserung der kutanen Wahrnehmung der Umwelt, vor allem zur Vermeidung einer zu hohen kutanen Reaktivtät und/oder zur Bekämpfung der durch die Umwelt bedingten kutanen Aggressionen und/oder des kutanen Stresses, insbesondere zur Bekämpfung von Verbrennungen, extremer Kälte und/oder Schmerz.

7. Extrakt für seine Verwendung nach Anspruch 6 zur Verbesserung der kutanen Thermosensibilität und/oder der Wahrnehmung des Schmerzes.

8. Extrakt für seine Verwendung nach Anspruch 6 zur Bekämpfung von Juckreiz und/oder Unbehaglichkeitsgefühlen.

9. Extrakt für seine Verwendung nach Anspruch 6 zur Vorbeugung oder Behandlung von intoleranter Haut.

10. Extrakt für seine Verwendung nach einem der vorangehenden Ansprüche, wobei der Peptid- und Osidextrakt aus Schisandafrucht besteht aus:
- 10% bis 50% Peptiden, und
- 10% bis 60% Gesamtzuckern,
wobei die Prozentsätze in Bezug auf das Gesamtgewicht des Peptid- und Osidextrakts ausgedrückt sind.

11. Extrakt für seine Verwendung nach einem der vorangehenden Ansprüche für topische Applikation.

## Claims

1. A peptide and oside extract of Schisandra fruit for use as a cutaneous neurosensory agent.

2. The extract for use according to claim 1, for improving cutaneous innervation in the basal state.

3. The extract for use according to claim 1 or 2, for improving scarring.

4. The extract for use according to claim 1 or 2, for improving touch perception, advantageously as a massage product.

5. The extract for use according to claim 1 or 2, for combating age-related loss of cutaneous innervation in the basal state, advantageously for preventing loss of cutaneous sensitivity and/or for combating thinning of the epidermis.

6. The extract for use according to claim 1, for improving cutaneous perception of the environment, notably for avoiding excessive cutaneous reactivity and/or for combating cutaneous attacks and/or cutaneous stress from the environment, more particularly for combating burns, extreme cold, and/or pain.

7. The extract for use according to claim 6, for improving cutaneous thermosensitivity and/or pain perception.

8. The extract for use according to claim 6, for combating itching and/or sensations of discomfort.

9. The extract for use according to claim 6, for preventing or treating intolerant skins.

10. The extract for use according to any one of the preceding claims, wherein said peptide and oside extract of Schisandra fruit consists of:
- 10% to 50% peptides, and
- 10% to 60% total sugars,
the percentages being expressed in relation to the total weight of said peptide and oside extract.

11. The extract for use according to any one of the preceding claims, for topical application.
